(19) Europäisches Patentamt
European Patent Office
Office européen des brevets

(11) **EP 2 030 023 B1**

(12) **EUROPÄISCHE PATENTSCHRIFT**

(45) Veröffentlichungstag und Bekanntmachung des Hinweises auf die Patenterteilung:
**17.04.2013 Patentblatt 2013/16**

(51) Int Cl.:
***G01N 33/68*** *(2006.01)*

(21) Anmeldenummer: **07764672.7**

(22) Anmeldetag: **15.06.2007**

(86) Internationale Anmeldenummer:
**PCT/EP2007/005299**

(87) Internationale Veröffentlichungsnummer:
**WO 2007/144194 (21.12.2007 Gazette 2007/51)**

(54) **IN VITRO MULTIPARAMETER-BESTIMMUNGSVERF AHREN ZUR DIAGNOSE UND FRÜHDIAGNOSE VON DEMENZERKRANKUNGEN**

IN VITRO MULTIPARAMETER DETERMINATION METHOD FOR THE DIAGNOSIS AND EARLY DIAGNOSIS OF DEMENTIA

PROCÉDÉ MULTIPARAMÈTRE DE DÉTERMINATION IN VITRO POUR LE DIAGNOSTIC ET LE DIAGNOSTIC PRÉCOCE DE MALADIES DEMENTIELLES

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **16.06.2006 DE 102006027818**

(43) Veröffentlichungstag der Anmeldung:
**04.03.2009 Patentblatt 2009/10**

(73) Patentinhaber: **B.R.A.H.M.S GmbH**
**16761 Hennigsdorf (DE)**

(72) Erfinder:
• **BERGMANN, Andreas**
**13465 Berlin (DE)**
• **SPARWASSER, Andrea**
**16761 Hennigsdorf (DE)**
• **HAMPEL, Harald**
**80331 München (DE)**

(74) Vertreter: **Andrae, Steffen et al**
**Andrae Flach Haug**
**Balanstrasse 55**
**81541 München (DE)**

(56) Entgegenhaltungen:
WO-A-2004/046181    WO-A-2004/059293
WO-A-2004/090546    WO-A-2005/078456
WO-A1-2007/131775

• **DATABASE MEDLINE [Online] US NATIONAL LIBRARY OF MEDICINE (NLM), BETHESDA, MD, US; Juni 1992 (1992-06), YOSHIZAWA T ET AL: "Cerebrospinal fluid endothelin-1 in Alzheimer's disease and senile dementia of Alzheimer type." XP002453611 Database accession no. NLM1407412**
• **ALBADALEJO M D ET AL: "Plasmatic determination of natriuretic peptides in demented patients" REVISTA DE NEUROLOGIA, JBT, BARCELONA, ES, Bd. 25, Nr. 139, März 1997 (1997-03), Seite 475, XP008082686 ISSN: 0210-0010 in der Anmeldung erwähnt**
• **ABDI FADI ET AL: "Detection of biomarkers with a multiplex quantitative proteomic platform in cerebrospinal fluid of patients with neurodegenerative disorders" JOURNAL OF ALZHEIMER'S DISEASE, IOS PRESS, AMSTERDAM, NL, Bd. 9, Nr. 3, August 2006 (2006-08), Seiten 293-348, XP009090146 ISSN: 1387-2877**

**Beschreibung**

Die vorliegende Erfindung betrifft ein neues *in vitro* Verfahren für die Diagnose und insbesondere Frühdiagnose von Demenzerkrankungen wie der Alzheimer Krankheit und deren Vorstufen, bei dem bei der Erstellung der Diagnose gleichzeitig mehrere auf Gefäße wirkende (vasotrope) bzw. blutdruckregulierende Substanzen als Biomarker, insbesondere Kombinationen von gefäßerweiternden (vasodilatorischen) und/oder gefäßverengenden (vasokonstriktorischen) physiologisch aktiven Peptiden, berücksichtigt werden.

Im Rahmen der vorliegenden Erfindung wird dabei der Begriff "Diagnose" als Oberbegriff für medizinische Bestimmungen gebraucht, denen je nach dem klinischen Zustand des Patienten (der untersuchten Person), bei dem die Bestimmung durchgeführt wird, unterschiedliche Fragestellungen zugrunde liegen können und die der Erkennung und im vorliegenden Falle insbesondere auch der Früherkennung, der Bestimmung des Schweregrads und der Verlaufsbeurteilung, auch der therapiebegleitenden Verlaufsbeurteilung, und der Prognose des zukünftigen Verlaufs einer Erkrankung dienen. Dabei ist im vorliegenden Zusammenhang von besonderer Bedeutung, dass eine Diagnose auch eine Negativdiagnose sein kann, bei der aufgrund der Nichtfeststellbarkeit bestimmter krankheitstypischer Merkmale, z.B. der Nichtnachweisbarkeit von mit der betreffenden Krankheit assoziierten Biomarkern oder Biomarkerkombinationen in einer Blutprobe eines Patienten, das Vorliegen einer bestimmten Erkrankung unwahrscheinlich gemacht wird.

Für die Negativdiagnose sind auch Biomarker von großem Wert, die bei mehreren verschiedenen Krankheiten erhöht gefunden werden können und daher allein, für sich genommen, noch keine positive Diagnose einer spezielle Krankheit ermöglichen - obwohl sie in der Regel bei Hinzuziehung weiterer klinischer oder biochemischer Parameter auch für die Positivdiagnose entscheidend sein können. Biomarkerkombinationen ermöglichen daher häufig Aussagen, die bei einer isolierten Bestimmung nur einzelner Biomarker oder Parameter nicht oder nicht mit der gleichen Wahrscheinlichkeit gemacht werden könnten.

Die Erkrankungen, um deren Diagnose es bei der vorliegenden Erfindung geht, sind sich eher langsam entwickelnde, chronische neurodegenerative Erkrankungen von nichtinfektiöser Ätiologie, nämlich Demenz-Erkrankungen.

Als Demenz-Erkrankungen (Dementia) werden generell Krankheiten bezeichnet, für die ein gemeinsames Merkmal der Verlust erworbener intellektueller Fähigkeiten, v.a. des Gedächtnisses, und des normalen Persönlichkeitsniveaus als Folge von Hirnschädigungen ist. Demenzerkrankungen sind in der Regel sich relativ langsam entwickelnde Krankheiten von chronischem Charakter. Treten Demenzerscheinungen vor dem hohen Alter im mittleren Lebensalter auf, werden sie als präsenile Demenz-Erkrankungen bezeichnet, und bei diesen unterscheidet man auf der Basis der für sie typischen Symptome und hirnpathologischen Veränderungen insbesondere die folgenden Erkrankungen bzw. Gruppen von Erkrankungen:

Die **Alzheimer Demenz (AD)** (Alzheimer Krankheit; Morbus Alzheimer) ist die häufigste neurodegenerative Demenz-Erkrankung, macht 2/3 aller Demenzfälle aus und stellt auch das praktisch wichtigste Anwendungsgebiet für die vorliegende Erfindung dar. AD zeichnet sich durch drei wichtige, allerdings erst post mortem mit Sicherheit feststellbare pathologische Merkmale aus: die Bildung von Amyloid-Plaques und neurofibrillären Bündeln sowie den Verlust an Nervenzellen (Übersicht s. (1); Literaturangaben in der Beschreibung in Form von Zahlen beziehen sich auf die der Beschreibung folgende Literaturliste). Amyloid-Plaques bestehen aus extraneuronalen Aggregaten des Amyloid-$\beta$-Proteins, während die Neurofibrillenbündel hauptsächlich Tau-Protein und Neurofilamente enthalten. Es wird vermutet, dass die Plaqueund Neurofibrillenbildung die Ursache für das Absterben von Nervenzellen ist.

Die wichtigsten Symptome von AD sind zunehmende Merkfähigkeits- und Denkstörungen bei relativ lang anhaltender Gemütsansprechbarkeit, wobei diese Symptome von weiteren weniger spezifischen Störungen begleitet werden, die die Abgrenzung der AD von anderen Demenzformen erschweren.

Beobachtungen an AD-Patienten und Patienten, die im Laufe ihrer langjährigen klinischen Beobachtung AD entwickelten, führten zur Formulierung von Kriterien für gegeneiander abgrenzbare Patientengruppen, die die ganze Breite von

(a) Personen ohne subjektive und objektive kognitive Störungen (die im Rahmen der vorliegenden Anmeldung die Kontrollgruppe repräsentieren), über

(b) Patienten, die über subjektiv empfundene kognitive Leistungseinbußen klagen, bei denen jedoch keine kognitiven Defizite festgestellt werden können (im Rahmen der vorliegenden Anmeldung ist das die Gruppe der "SKS"-Patienten, wobei "SKS" für "subjektive kognitive Störungen" steht), weiter über

(c) Patienten, bei denen leichte kognitive Störungen feststellbar sind und bei denen dann, wenn keine anderen demenzverursachenden Erkrankungen vorliegen, die Diagnose "möglicherweise AD" ("mgl AD") gestellt wird (im Rahmen der vorliegenden Anmeldung ist das die Gruppe "LKS mgl AD", wobei "LKS" für "leichte kognitive Störungen" steht) bis hin

(d) zur Gruppe der Patienten mit dem typischen Bild erheblicher kognitiver Störungen, die schleichend begonnen haben und langsam progredieren, für die dann, wenn andere Demenzursachen ausgeschlossen werden können, die Diagnose "wahrscheinlich AD" gestellt wird (im Rahmen der vorliegenden Anmeldung ist das die Gruppe "ws AD", wobei die Abkürzung für "wahrscheinlich Alzheimer" steht),

abdecken.

Bezüglich der Zuordnung von Personen bzw. Patienten mit subjektiven und/oder objektiven kognitiven Störungen zu verschiedenen Gruppen wird ergänzend verwiesen auf (2), (3), (4) und (5).

Die **Demenz mit Lewy-Körperchen** (dementia with lewy-bodies: DLB) ist nach der Alzheimer Demenz die zweithäufigste Ursache für eine demenzielle Erkrankung. Neuropathologisch ist die DLB durch das Auftreten von sogenannten Lewy-Körperchen im Hirnstamm und im Cortex charakterisiert. Diese LewyKörperchen bestehen überwiegend aus Aggregaten des präsynaptischen Proteins ($\alpha$-Synuclein) und Ubiquitin. Die LewyBody-Pathologie kann in unterschiedlichem Ausmaß mit Alzheimer und Parkinson-typischen neuropathologischen Verbänderungen assoziiert sein. So kommt es auch bei der DLB zur Bildung von beta-Amyloid und senilen Plaques, jedoch nicht zu Neurofibrillenbündeln (Übersicht s. (6)). Lewy-Körperchen sind auch im Gehirn von Patienten mit Morbus Parkinson vorhanden, wenn auch in einer unterschiedlichen Verteilung.

Kernsymptome von DLB sind eine progrediente kognitive Störung, Verwirrtheitsepisoden mit fluktuierender Aufmerksamkeits- und Bewusstseinslage, Parkinsonismus, häufige Stürze und Synkopen (anfallsartige, kurz dauernde Bewusstlosigkeit). Die Sensitivität und Spezifität der diagnostischen Kriterien sind durchgehend hoch bezüglich der Spezifität, aber zum Teil sehr niedrig bezüglich der Sensitivität. Das bedeutet, dass die DLB im klinischen Alltag häufig nicht diagnostiziert wird.

Die **Frontotemporale Demenz (FTD)** wird auch als Pick'sche Krankheit bezeichnet und macht ca. 20% der präsenilen Demenzerkrankungen aus. FTD ist teilweise genetisch bedingt und zählt zu den sogenannten Tauopathien, die sich durch eine Über- oder Unterexpression eines Tauprotein-Subtyps bzw. durch die Expression eines mutierten Tauproteins auszeichnen. Neuropathologisch kommt es zu einer lokalen Atrophie des frontalen und/oder temporalen Cortex sowie der Substantia Nigra und der Basalganglien. Dies hat unterschiedlich ausgeprägte Sprachstörungen, eine Wesensänderung sowie Verhaltensauffälligkeiten zur Folge. Insgesamt ist die FTD mit einer Sensitivität von 93% bei einer Spezifität von nur 23% unterdiagnostiziert, wobei die AD die häufigste Fehldiagnose darstellt.

Unter dem Begriff **vaskuläre Demenz (vascular dementia; VAD)** werden Erkrankungen zusammengefasst, bei denen eine Demenz aufgrund von Durchblutungsstörungen im Gehirn ausgelöst wird. Es gibt unterschiedliche Typen der VAD, von denen die Multi-Infarkt-Demenz (MID) und die subcorticale VAD (auch als Binswanger'sche Erkrankung bezeichnet) die häufigsten Formen darstellen.

Bei der Binswanger'schen Erkrankung handelt es sich um eine langsam progrediente demenzielle Entwicklung, die pathologisch durch cerebrovasculäre Läsionen in der weißen Hirnsubstanz charakterisiert ist. Klinisch resultiert dies in Verhaltensauffälligkeiten wie Agitation, Reizbarkeit, Depression und Euphorie sowie einer leichten Gedächtnisstörung.

Die Multi-Infarkt-Demenz entsteht allmählich als Folge von mehreren kleinen Schlaganfällen, auch als transiente ischämische Attacken (TIA) bezeichnet, die zum Untergang von Hirngewebe im Cortex und/oder subcortikalen Arealen führten. Die Schlaganfälle können auch gänzlich unbemerkt geblieben sein, die Demenz ist in diesem Falle die erste spürbare Folge. Bei Vorliegen einer MID kommt es zur stufenweisen Abnahme kognitiver Fähigkeiten, verbunden mit schweren Depressionen, Stimmungsschwankungen und Epilepsie.

Eine Diagnose auf Demenz-Erkrankungen erfolgt heutzutage überwiegend auf der Basis neuropsychologischer Untersuchungen und der Beobachtung der Krankheitsentwicklung und ihres Verlaufs, unter Heranziehung von Ausschlusskriterien für bestimmte Demenzformen. Diese Untersuchungen liefern in sehr vielen Fällen mehrdeutige Ergebnisse, die die o.g. Zahlen für die unterdiagnostizierten Demenzformen oder unrichtig diagnostizierten Fälle erklären. Die krankheitstypischen Gehirnveränderungen können an lebenden Patienten naturgemäß nicht direkt festgestellt werden, und apparatemedizinische Untersuchungen der Gehirnfunktionen mittels z.B. Röntgen- oder Kernspin-Tomographie sind aufwändig und teuer.

Es wäre daher wünschenswert, die Erkennung und insbesondere Früherkennung von Demenzerkrankungen durch die Messung von aussagekräftigen Biomarkern, die mit Hilfe relativ einfacher Testverfahren z.B. in einer Blutprobe (Serumprobe, Plasmaprobe) eines Patienten bestimmt werden können, ergänzen und dadurch erheblich verbessern zu können.

Für die Alzheimer-Diagnostik veröffentlichten das Ronald und Nancy Reagan Institut der Alzheimer Association und die NIA-Working Group Richtlinien für die Kriterien, die an einen idealen Biomarker zur Detektion der AD gestellt werden (7). Folgende Kriterien sollen im Idealfall durch den Biomarker erfüllt werden:

1. Er sollte hirnspezifisch sein und ein fundamentales Merkmal der Neuropathologie dieser Erkrankungen detektieren.
2. Die diagnostische Sensitivität und die Spezifität von mindestens 80% sollte gegeben sein.

3. Die krankheitsspezifische Veränderung des Biomarkers sollte sich in einem möglichst frühen Stadium der Erkrankung manifestieren, um mit geeigneten Therapiemaßnahmen beginnen zu können (8).

Bis jetzt gibt es jedoch keinen Biomarker, der im klinischen Alltag im Blut oder der cerebrospinalen Flüssigkeit mit ausreichender Sicherheit zur Früh- und Differentialdiagnose von AD herangezogen werden könnte und alle o.g. Kriterien erfüllt. Aktuell werden verschiedene potentielle Markerkandidaten untersucht, darunter Inflammationsmarker wie IL-6 und $TNF\alpha$, Marker für oxidativen Stress wie 3-Nitrotyrosin, sowie Marker, die mit charakteristischen pathologischen Veränderungen der AD assoziiert sind, wie Amyloid $\beta$, das einen Hauptbestandteil der Amyloid-Plaques darstellt, und das Tau-Protein, das einen wesentlichen Bestandteil der Neurofibrillenbündel darstellt (vgl. die Übersicht in (8); (9)).

Es besteht ein aktueller Bedarf nach ergänzenden, valide Laborbefunde liefernden Untersuchungsmethoden, die auf einer Bestimmung von als Biomarker für Demenzerkrankungen, insbesondere für die Alzheimer Demenz (AD), geeigneten Substanzen in Blut- bzw. Plasmaproben beruhen und bei Personen, bei denen der Verdacht auf Vorliegen einer Demenzerkrankung, insbesondere von AD, besteht, unterstützend für eine frühe Positivdiagnose und/oder für eine negative Ausschlussdiagnose geeignet sind.

Die vorliegende Erfindung stellt eine solche Untersuchungsmethode in Form eines *in vitro* Multiparameter-Verfahrens zur Erkennung und Früherkennung, zur Bestimmung des Schweregrads und zur Verlaufsbeurteilung und Prognose bereit, bei dem man

in einer biologischen Flüssigkeit einer Person, die an subjektiven oder objektiv nachweisbaren kognitiven Störungen leidet, die Konzentrationen von mindestens zwei Analyten bestimmt, die aus der Gruppe der vasotropen Peptide ausgewählt sind,

die erhaltenen personenspezifischen Messwerte für die jeweiligen Konzentrationen der mindestens zwei bestimmten Analyten rechnerisch zu einem personenpezifischen komplexen Bezugswert kombiniert und

auf der Basis des ermittelten personenspezifischen komplexen Bezugswerts Schlüsse hinsichtlich des Vorliegens einer dementiellen Erkrankung bei der Person, oder einer für diese typischen Frühform, oder hinsichtlich des Verlaufs der Erkrankung und/oder des Erfolgs der Bemühungen zu ihrer Abmilderung oder Verhinderung zieht.

Die Gruppe der vasotropen Peptide umfasst eine erste Untergruppe der vasodilatorischen Peptide und eine zweite Untergruppe der vasokonstriktorischen Peptide.

Bei der rechnerischen Auswertung kombiniert man personenspezifische Messwerte für die Konzentrationen der gemessenen vasotropen Peptide derselben Untergruppe gleichrangig, vorzugsweise durch Multiplikation, und personenspezifische Messwerte für Konzentrationen vasotroper Peptide verschiedener Untergruppen vorzugsweise durch Division.

Die Konzentrationen der vasotropen Peptide bestimmt man vorzugsweise auf dem Wege der Bestimmung der Konzentrationen von aus zugeordneten Propeptid-Vorläufern freigesetzten physiologisch inaktiven Propeptidfragmenten, die nicht das eigentliche vasotrope Peptid sind, in den untersuchten biologischen Proben, insbesondere in Serum oder Plasma.

Als vasodilatorische Peptide bestimmt man vorzugsweise die natriuretischen Peptide ANP, BNP und/oder CNP und/oder das Peptid Adrenomedullin und/oder das Peptid CGRP (calcitonin gen related peptide), und als vasokonstriktorische Peptide ein Endothelin, insbesondere Endothelin 1 (ET-1). Das erfindungsgemäße Verfahren kann jedoch auch dadurch modifiziert werden, dass man zusätzlich, andere vasotrope Analyten, die den beiden o.g. Untergruppen zugeordnet werden können, bestimmt oder mitbestimmt. Es können in diesem Zusammenhang ergänzend genannt werden einerseits die Peptide des Renin-Angiotensin-Aldosteron (RAA) Systems, insbesondere das vasokonstriktorische Peptid Angiotensin II, sowie Arginin-Vasopressin (AVP), Substanz P (SP), sowie andererseits die vasodilatorischen Peptide $CGRP_{1-37}$, Amylin (IAPP), Endothelin-3 (ET-3) und das vasoaktive Intestinalpeptid (VIP). Ferner ist auch eine Mitberücksichtigung von weiteren Nicht-Peptid-Analyten wie NO (z.B. bestimmbar als Nitrit oder Nitrat) zu nennen. Zur Ergänzung sowie im Hinblick auf weiterführende Literatur zu den genannten einzelnen potentiellen Analyten wird verwiesen auf (10) und (11). Auf die (10) bzw. (11) entnehmbaren bekannten weiteren Paare von vasotropen Substanzen, die mit gegenläufiger Wirkung am gleichen physiologischen Steuerungsvorgang beteiligt sind, wird ergänzend ausdrücklich verwiesen.

Die Bestimmung der jeweiligen Analyten in einer biologischen Flüssigkeit erfolgt vorzugsweise in Vollblut, Serum oder Plasma.

Die Konzentration von ANP wird vorzugsweise als Konzentration eines proANP-Fragments mit Hilfe eines Assays der Anmelderin bestimmt, der einen mittleren Abschnitt des proANP (MR-proANP) erkennt. Ein solcher Assay ist beschrieben in EP 1 562 984 B1 bzw. in (12).

Die Konzentration von Adrenomedullin (ADM) wird vorzugsweise als Konzentration eines midregionalen proADM-Fragments (MR-proADM) bestimmt, das die Aminosäuren 45-92 des Prä-Proadrenomedullins umfasst. Ein geeigneter Assay ist beschrieben in EP 1 488 209 B1 bzw. WO 2004/090546 bzw. in (13).

Die Konzentration von Endothelin 1 (ET-1) wird vorzugsweise als Konzentration eines C-terminalen ET-1-Fragments (CT-proET-1) bestimmt, das die Aminosäuren 168-212 des Prä-Proendothelins 1 umfasst. Ein geeigneter Assay ist beschrieben in 1 564 558 B1 bzw. WO 2005/078456 und in (14).

Die Bestimmung der mindestens zwei Analyten führt man vorzugsweise mit Hilfe immundiagnostischer Bestimmungsverfahren in Form von Immunoassays vom Sandwichtyp durch, z.B. mit einem der oben erwähnten Assays der Anmelderin.

Gemäß einer bevorzugten Ausführungsform des erfindungsmäßen Verfahrens teilt man zur Ermittlung des patientenspezifischen Bezugswerts den Messwert für die Konzentration eines der oben genannten vasodilatorischen Peptide, oder den durch rechnerische Multiplikation von wenigstens zwei Messwerten für die Konzentrationen von wenigstens zwei vasodilatorischen Peptiden erhaltenen Wert, durch die Konzentration für das vasokonstriktorische Peptid ET-1.

Die neurodegenerative Erkrankung, deren verbesserte Diagnose Gegenstand der vorliegenden Erfindung ist, ist eine Demenzerkrankung, die ausgewählt ist aus einer Gruppe, die die Alzheimer Demenz (AD), Demenz mit Lewy-Körperchen (DLB), frontotemporale Demenz (FTD) und verschiedene Formen der vaskulären Demenz (VD) umfasst, wobei das Verfahren vorzugsweise im Rahmen der Alzheimer-Diagnostik zur Erkennung von Frühformen der Alzheimer Demenz (AD) durchgeführt wird.

Die erfindungsgemäße *in vitro* Multiparameter-Bestimmung kann man bei einer klinischen Routineanwendung in größerem Maßstab zweckmäßiger Weise auch als Simultanbestimmung mittels einer Chiptechnologie-Messvorrichtung oder im Rahmen einer sog. Point-of-Care (POC)-Bestimmung mit einer immunchromatographischen Messvorrichtung durchführen. Die Ermittlung und Auswertung des komplexen Messergebnisses der Multiparameter-Bestimmung erfolgt zweckmäßiger Weise mit Hilfe eines geeigneten Computerprogramms.

Wenn in dieser Anmeldung der Begriff "Konzentration" verwendet wird, meint dieser Begriff nicht, im Sinne einer einengenden Gleichsetzung, nur die in der biologischen Probe messbaren stationären Konzentration des eigentlichen vasotropen Peptids.

Die wichtigsten im Rahmen der vorliegenden Erfindung diskutierten, pathophysiologisch freigesetzten vasotropen Peptide liegen nur zu einem geringeren Teil frei bzw. unbehindert messbar in biologischen Flüssigkeiten vor. Wesentliche Teile der pathophysiologisch freigesetzten vasotropen Peptide werden der biologischen Flüssigkeit durch Bindung an Rezeptoren und andere Membran- oder Gefäßstrukturen rasch entzogen und/oder abgebaut.

Die Messung von inaktiven, aus den gleichen Vorläufer-Propeptiden gebildeten Co-Peptiden, wie sie gemäß der vorliegenden Erfindung unter Verwendung der hierin genannten Assays der Anmelderin bevorzugt erfolgt, spiegelt, anders als die momentane Konzentration in einer biologischen Flüssigkeit, die Freisetzung der vasotropen Peptide im Sinne von "Wirkkonzentrationen" über einen längeren Zeitabschnitt wieder und gestattet eine indirekte Miterfassung auch gebundener oder schnell abgebauter Anteile des vasoaktiven freigesetzten Peptids. Das führt in Verbindung mit der höheren Stabilität solcher Co-Peptide zu höheren messbaren Absolutkonzentrationswerten für den zu bestimmenden Analyten in der biologischen Flüssigkeit, z.B. in Serum oder Plasma.

Bei Erkrankungen mit einem eher chronischen Verlauf ohne plötzliche Verschlechterungen oder Verbesserungen des Zustands des Patienten, wie es bei Demenzerkrankungen die Regel ist, besteht z.B. eine hohe Wahrscheinlichkeit dafür, dass sich bezüglich der verschiedenen krankheitsrelevanten Analyten ein nur wenig variabler, sich nur langsam verändernder stationärer Gesamtzustand ausbildet. In einem solchen Falle sollten die stationär in der biologischen Flüssigkeit des Patienten messbaren Konzentrationen eines aktiven Analyten, im vorliegenden Falle eines vasotropen Peptids, im wesentlichen proportional zu den über längere Zeiträume pathophysiologisch freigesetzten Mengen des gleichen Analyten sein, wie sie in Form des physiologisch inaktiven Co-Peptids gemessen werden können. Das bedeutet, dass sich die bei der bevorzugten Multiparameter-Bestimmung inaktiver Co-Peptide gemäß der vorliegenden Erfindung festgestellbaren Abweichungen von den Kontrollwerten Gesunder, sowie der krankheitstypische Gang dieser Abweichungen, auch in den in der Regel niedrigeren stationären Konzentrationen der aktiven Analyten widerspiegeln sollten, so dass der konkreten Wahl der gemessenen "Analytenkonzentrationen" kein entscheidender qualitativer Einfluss auf die diagnostische Auswertung zukommen sollte.

Der vorliegenden Erfindung liegen Überlegungen der Erfinder zugrunde, zur Verbesserung der Diagnostik von Demenz-Erkrankungen bei der Erkenntnis anzusetzen, dass die bekannten, eingangs näher erläuterten Formen der Demenz auch - in unterschiedlichem Ausmaß - von entzündlichen (inflammatorischen) Prozessen und Endothelschäden begleitet sind, die als wesentlich für die Entwicklung, die Symptome und den Verlauf der Demenzerkrankungen angesehen werden, weshalb neurodegenerative Erkrankungen auch als neuroinflammatorische Erkrankungen angesehen werden können.

So ist Morbus Alzheimer u.a. durch das Auftreten chronischer lokaler Entzündungsreaktionen im Gehirn unter Beteiligung von verschiedenen inflammatorischen Proteinen wie Komplement-Faktoren, Akutphasen-Proteinen und proinflammatorischen Cytokinen gekennzeichnet (9).

Inflammatorische Prozesse spielen auch eine Rolle bei der Entstehung von vaskulären Demenzen (VAD). Die Level von TNF$\alpha$, TGF$\beta$, IL-6 und GM-CSF (Granulozyten-Makrophagen-stimulierender Faktor) sind bei Patienten mit VAD deutlich erhöht (15, 16) .

Auch bei DLB scheinen inflammatorische Prozesse eine Rolle zu spielen. So ist die Zahl der aktivierten Mikroglia-Zellen im Gehirn von Patienten mit DLB erhöht, und proinflammatorische Cytokine wie TNFα werden in bestimmten Hirnregionen wie der Amygdala und dem Hippocampus überexprimiert.

Für das Auftreten inflammatorischer Reaktionen im Gehirn von FTD-Patienten gibt es dagegen nur vereinzelte Hinweise.

Ausgehend (i) von der Hypothese, dass die mit Demenzerkrankungen verbundenen neuroinflammatorischen Prozesse zu Endothelstress und Durchblutungsstörungen, insbesondere auch zu Mikrozirkulationsstörungen des Gehirns führen, und (ii) dass insoweit eine Ähnlichkeit mit cardiovasculären Erkrankungen besteht, die mit Durchblutungsstörungen bzw. Störungen der Mikrozirkulation des Herzgewebes verbunden sind, sowie (iii) von orientierenden analytischen Befunden, die zeigen, dass bei solchen cardiovasculären Erkrankungen eine erhöhten Bildung verschiedener vasotroper Peptide feststellbar ist, sowie schließlich (iv) unter Nutzung der verbesserten analytischen Möglichkeiten zur Bestimmung der Konzentrationen verschiedener vasotroper Peptide in zuverlässiger und in klinisch valider Form mit Hilfe neuartiger Immunoassays der Anmelderin, bei denen man eine Bestimmung physiologisch inaktiver Peptidfragmente der Propeptid-Vorläufer von vasotropen Peptiden durchführt, untersuchten die Erfinder die Frage, ob sich auch bei Patienten mit kognitiven Störungen unterschiedlichen Ausmaßes, die ansonsten an keiner bekannten, mit einer erhöhten Produktion vasotroper Peptide verbundenen Erkrankung litten, im Plasma Hinweise auf erhöhte Konzentrationen solcher Peptide finden lassen.

Dabei zeigte sich bei ersten Untersuchungen der Anmelderin, dass bei Patienten mit verschiedenen Demenzerkrankungen, insbesondere bei Patienten mit Alzheimer-Demenz (AD-Patienten), die vasodilatorischen Peptide ANP, BNP und CNP sowie auch Adrenomedullin (ADM) signifikant erhöht gefunden werden. Diese analytischen Befunde sind Gegenstand der noch nicht veröffentlichten älteren deutschen Patentanmeldungen der Anmelderin mit den Aktenzeichen 10 2005 036 094.7 vom 01.08.2005 bzw. 10 2006 023 175.9 vom 17.05.2006. Soweit in der vorliegenden Anmeldung eine Bestimmung von ANP (als MR-proANP) bzw. von ADM (als MR-proADM) in Patientenplasmen erfolgt, erfolgt eine solche Bestimmung so wie in den genannten älteren Patentanmeldungen der Anmelderin beschrieben wird.

Dass zwischen Adrenomedullin-Produktion und Mikrozirkulation ein Zusammenhang bestehen kann, ist aus (17) bekannt. Demenzerkrankungen werden in diesem Zusammenhang aber nicht diskutiert.

Soweit in der Literatur Versuche der Messung natriuretischer Peptide bei Personen beschrieben wurden, die demenzartige Symptome zeigten, war keine signifikante Korrelation festgestellt worden (18, 19).

Die nachfolgend im experimentellen Teil beschriebenen Messergebnisse in EDTA-Plasmaproben von 60 augenscheinlich gesunden Normalpersonen (symptomfreien Kontrollen) und 196 Patienten mit leichten bis schweren kognitiven Störungen gemäß den eingangs beschrieben Gruppen (b) bis (d) ergab erstmals eine klare, diagnostisch signifikante Korrelation zwischen den für z.B. MR-proADM und insbesondere MR-proANP gefundenen Konzentrationen und der Schwere der Demenzsymptome in Form kognitiver Störungen, wobei die gemessenen Konzentrationen in signifikanter Weise mit der Schwere der Störungen und damit der AD-Vorstufen korrelierten und damit zur Unterscheidung der verschiedenen Patientengruppen beitragen können.

Eine parallele Bestimmung von CT-ET-1 ergab für Demenzpatienten gegenüber gesunden Normalpersonen (Kontrollen) erniedrigte Messwerte, die jedoch den obigen Gang nicht klar widerspiegelten wie z.B. die Messwerte für MR-proANP.

Es stellte sich gemäß der vorliegenden Erfindung jedoch heraus, dass man dann, wenn man die Messwerte für z.B. ANP (MR-proANP) und/oder ADM (MR-proADM) in geeigneter Weise mit denen einer Messung von CT-ET-1 kombinierte, und zwar in dem Sinne, dass man die für einen bestimmten Patienten gemessenen Werte für die vasodilatorischen Peptide MR-proANP oder MR-proADM oder ggf. den durch Multiplikation der Werte für MR-proANP und MR-proADM erhaltenen kombinierten Wert auf die für jeweils den gleichen Patienten gemessenen Werte für das vasokonstriktorische Peptid ET-1 (gemessen als CT-proET-1) bezog, indem man die Werte für die gemessenen Konzentrationen der vasodilatorischen Peptide bzw. für deren mathematisches Produkt durch die Werte für CT-ET-1 teilte, eine weitere deutliche Verbesserung der Signifikanz der Gesamtmessung im Sinne einer Multiparameter-Messung erhielt.

Indem man somit die patientenspezifischen Werte für die vasodilatorischen Peptide zu Auswertungszwecken nicht nur auf einen externen Cut-Off-Wert bezog, der ein statistisches Ergebnis der Messung entsprechender Analyten-Konzentrationen bei einem größeren heterogenen Kollektiv von Patienten und Kontrollen darstellt, sondern zuerst auf einen patientenspezifischen internen Bezugswert in Form eines Messwerts für mindestens ein beim gleichen Patienten gemessenes relevantes vasokonstriktorisches Peptid, hierin insbesondere für CT-proET-1, der als Ausdruck eines internen und individuell variablen Patientenstatus oder -standards angesehen werden kann, erhöht sich die Signifikanz der Messung der vasodilatorischen Peptide noch einmal erheblich.

Setzt man die bei einer solchen Auswertung erhaltenen komplexen Bezugswerte zu entsprechenden, z.B. bei einem Kollektiv von Kontrollen oder von Patienten mit anderen Erkrankungen oder mit einer anderen Schwere der Erkrankung erhaltenen Durchschnittswerten in Beziehung, erhöht sich insbesondere die Sensitivität der Gesamtbestimmung erheblich.

Dieser Befund wird nachfolgend im experimentellen Teil unter Bezugnahme auf mehrere Figuren und zwei Tabellen noch näher erläutert.

Obwohl die Untersuchungen bisher auf Plasmaproben von Patienten beschränkt waren, die Anzeichen von Vorstufen von AD zeigten bzw. für die die Diagnose "wahrscheinlich Alzheimer" gestellt worden war, gehen die Erfinder davon aus, dass - möglicherweise mit unterschiedlichen typischen Konzentrationsbereichen - auch bei anderen neuroinflammatorischen Demenzformen, insbesondere bei vaskulärer Demenz (VAD) und Demenz mit Lewy-Körperchen (DLB), charakteristische Veränderungen der Konzentrationen vasotroper Peptide in Patientenplasmen feststellbar sein könnten.

Das für die im experimentellen Teil beschriebenen Messungen eingesetzte Bestimmungsverfahren für MR-proANP, MR-proADM, CT-pro-ET-1 in Patientenplasmen erfolgte mit den weiter oben erwähnten, in der dort genannten Literatur beschriebenen Assays der Anmelderin, die alle ihrem Wesen nach nichtkompetitive immunoluminometrische Sandwichassays darstellen.

Auf die allgemeinen Ausführungen zur Problematik der Bestimmung von ANP bzw. ADM bzw. ET-1 in Patientenproben und die Erläuterungen zur Assaydurchführung in den genannten Patentschriften bzw. den Publikationen (12, 13, 14) wird zur Ergänzung der Ausführungen in der vorliegenden Anmeldung ausdrücklich Bezug genommen.

Für den Fall einer zusätzlichen, ergänzenden Bestimmung der Konzentrationen des vasokonstriktorisch wirkenden Peptids Arginin-Vasopressin (AVP) ist die Verwendung eines Assays empfehlenswert, mit dem das Propeptid-Fragment Copeptin bestimmt wird und der in näheren Einzelheiten beschrieben wird in WO 2006/018315 bzw. in (20).

Nachfolgend wird die Erfindung anhand von Messergebnissen und 5 Figuren noch näher erläutert.

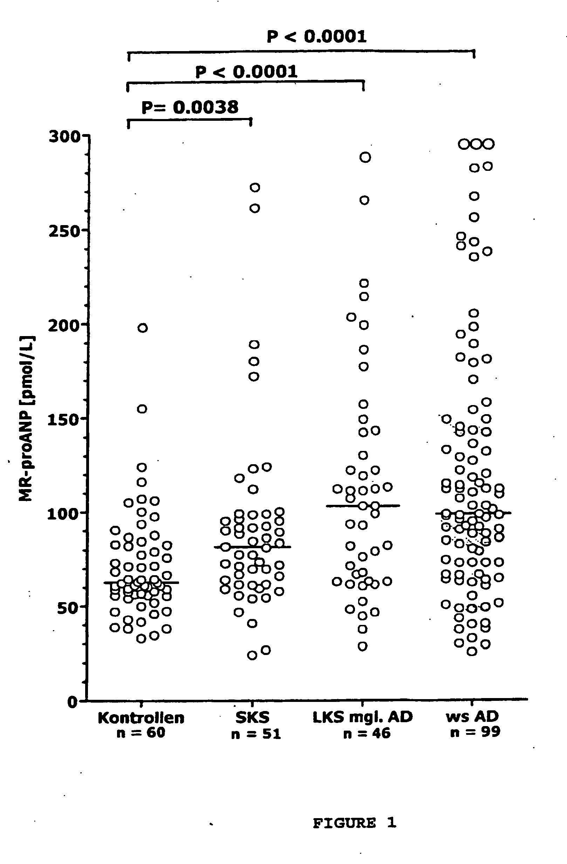

Figur 1     zeigt die Ergebnisse der Messung der MR-proANP-Konzentrationen in EDTA-Plasmen von 60 gesunden Kontrollpersonen, und von 196 Patienten mit kognitiven Störungen verschiedener Schwere, die den o.g. Gruppen (b), (c) und (d), d.h. den Gruppen "SKS" (51 Patienten), "LKS mgl AD" (46 Patienten) und "ws AD" (99 Patienten) entsprachen.

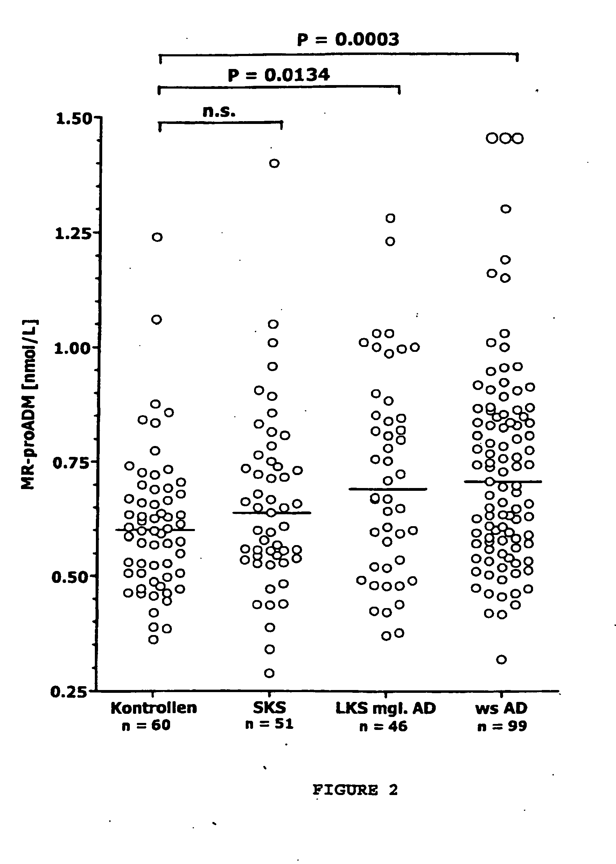

Figur 2     zeigt die Ergebnisse der Messung der MR-proADM-Konzentrationen in EDTA-Plasmen von 60 gesunden Kontrollpersonen, und von 196 Patienten mit kognitiven Störungen verschiedener Schwere, die den o.g. Gruppen (b), (c) und (d), d.h. den Gruppen "SKS" (51 Patienten), "LKS mgl AD" (46 Patienten) und "ws AD" (99 Patienten) entsprachen.

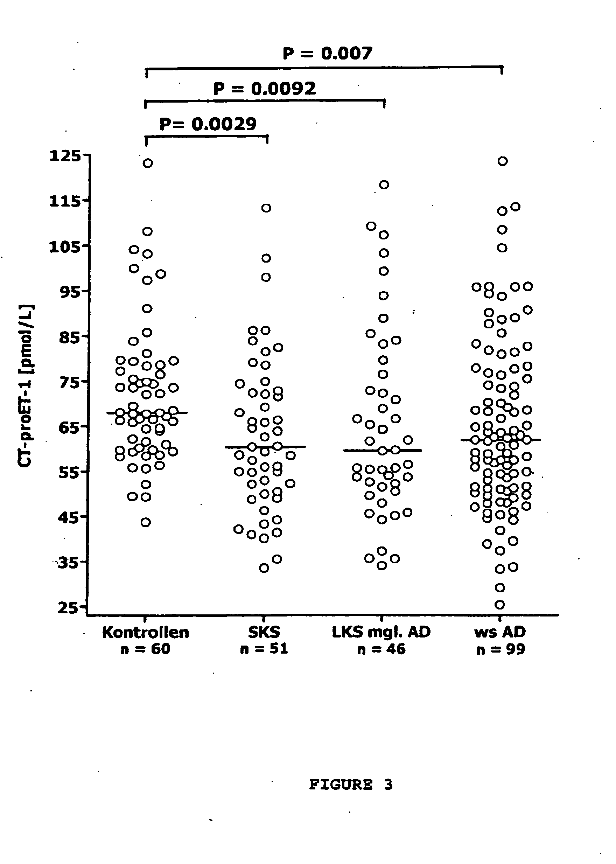

Figur 3     zeigt die Ergebnisse der Messung der CT-proET-1-Konzentrationen in EDTA-Plasmen von 60 gesunden Kontrollpersonen, und von 196 Patienten mit kognitiven Störungen verschiedener Schwere, die den o.g. Gruppen (b), (c) und (d), d.h. den Gruppen "SKS" (51 Patienten), "LKS mgl AD" (46 Patienten) und "ws AD" (99 Patienten) entsprachen.

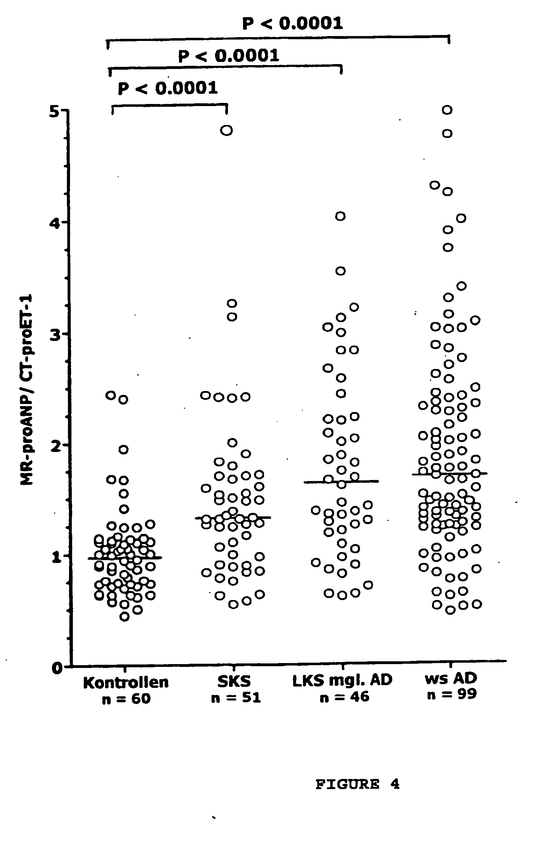

Figur 4     zeigt die Ergebnisse einer gemeinsamen rechnerischen Auswertung der in Figur 1 gezeigten Messergebnisse für die MR-proANP-Konzentrationen, wenn diese durch Quotientenbildung auf die in Figur 3 gezeigten Messergebnisse für die CT-proET-1 Konzentrationen bei den gleichen individuellen Patienten bezogen wurden, und zwar wiederum von 60 gesunden Kontrollpersonen, und von 196 Patienten mit kognitiven Störungen verschiedener Schwere, die den o.g. Gruppen (b), (c) und (d), d.h. den Gruppen "SKS" (51 Patienten), "LKS mgl AD" (46 Patienten) und "ws AD" (99 Patienten) entsprachen.

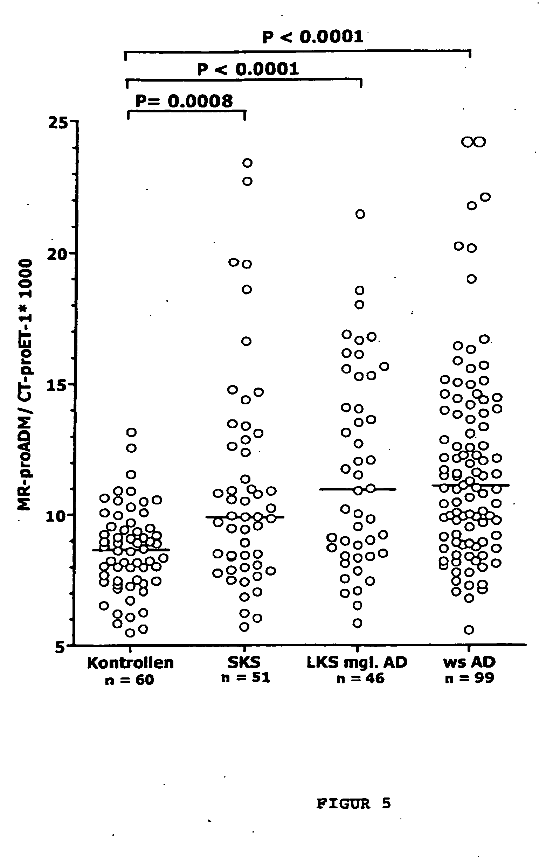

Figur 5     zeigt die Ergebnisse einer gemeinsamen rechnerischen Auswertung der in Figur 2 gezeigten Messergebnisse für die MR-proADM-Konzentrationen, wenn diese durch Quotientenbildung auf die in Figur 3 gezeigten Messergebnisse für die CT-proET-1 Konzentrationen bei den gleichen individuellen Patienten bezogen wurden, und zwar wiederum von 60 gesunden Kontrollpersonen, und von 196 Patienten mit kognitiven Störungen verschiedener Schwere, die den o.g. Gruppen (b), (c) und (d), d.h. den Gruppen "SKS" (51 Patienten), "LKS mgl AD" (46 Patienten) und "ws AD" (99 Patienten) entsprachen.

## Experimenteller Teil

### Assavbeschreibunq

Die Messung von MR-proANP im Plasma erfolgte mit einem immunoluminometrischen Sandwichassay im wesentlichen so wie im experimentellen Teil der o.g. WO 2004/046181 bzw. in (12) beschrieben wird.

Die Messung von MR-proADM im Plasma erfolgte mit einem immunoluminometrischen Sandwichassay im wesentlichen so wie im experimentellen Teil der o.g. WO 2004/090546 bzw. in (13) beschrieben wird.

Die Messung von CT-proET-1 im Plasma erfolgte mit einem immunoluminometrischen Sandwichassay im wesentlichen so wie im experimentellen Teil der o.g. WO 2005/078456 bzw. in (14) beschrieben wird.

Messung von MR-proANP, MR-proADM und CT-pro-ET-1 im Plasma von gesunden Kontrollen und Patienten mit kognitiven Störungen verschiedenen Schweregrads

Zur Ermittlung eines Bezugswerts für die Konzentration des jeweiligen Analyten wurde eine Messung in EDTA-Plasmen von 60 symptomfreien Kontrollpersonen durchgeführt, die weder Symptome kognitiver Störungen zeigten noch an irgendeiner anderen erkennbaren Erkrankung (cardiovasculäre Erkrankungen; schwere Infektion oder Entzündung) litten, für die bekannt ist, dass bei ihr die o.g. Biomarker-Analyten erhöht gemessen werden können. Für die Kontrollgruppe wurden Mittelwerte (Mediane) für die gemessene Konzentrationen wie folgt ermittelt:

$$
\begin{aligned}
\text{MR-proANP} &= 62{,}3 \text{ pmol/L} \\
\text{MR-proADM} &= 0{,}60 \text{ nmol/L} \\
\text{CT-proET-1} &= 68{,}0 \text{ pmol/L}
\end{aligned}
$$

Als Patientenkollektiv dienten Patienten mit Demenzerscheinungen in Form kognitiver Störungen verschiedener Schwere, aufgrund derer eine Zuordnung der einzelnen Patienten zu einer der o.g. Gruppen (b), (c) bzw (d) erfolgt war.

Die gemessenen MR-proANP-, MR-proADM- bzw. CT-proET-1-Konzentrationen im Plasma von gesunden Kontrollen und Patienten mit kognitiven Störungen sind in den Figuren 1 bis 3 gezeigt. Die Figuren 4 und 5 zeigen die Werte, die man erhält, wenn man die MR-proANP- bzw. MR-proADM-Konzentrationen auf die zugehörigen CT-proET-1-Konzentrationen bezieht (durch die CT-proET-1-Konzentrationen teilt).

Die ermittelten Zahlenwerte in Form der sog. Mediane für die verschiedenen Patientengruppen sind in Tabelle 1 gezeigt.

### TABELLE 1

| MARKER bzw. MARKER-KOMBINATION | Median Kontrollen | Median SKS | Median LKS mgl AD | Median ws AD |
|---|---|---|---|---|
| MR-proANP (pmol/L) | 62,3 | 81,5 | 103,0 | 98,8 |
| MR-proADM (nmol/L) | 0,60 | 0,64 | 0,69 | 0,71 |
| CT-proET-1 (pmol/L) | 68,0 | 60,40 | 59, 5 | 61,8 |
| MR-proADM/- CTproET-1 x 1000 | 8,67 | 9,91 | 10,96 | 11,10 |
| MR-proANP/ CT-proET-1 | 0,97 | 1,32 | 1,64 | 1,69 |

Die aus den Messdaten gemäß den Figuren 1 bis 5 unter Verwendung der jeweils angegebenen Werte für den Cut-off errechneten Spezifitäten und Sensitivitäten, und zwar für die verschiedenen Patientengruppen, sind in Tabelle 2 gezeigt, in die zusätzlich noch diejengen Werte aufgenommen wurden, die man erhält, wenn man das Produkt der Konzentrationen für MR-proANP und denen für MR-proADM bildet (MR-proANP x MR-proADM) bzw. wenn man dieses Produkt zusätzlich durch die zugehörigen CT-proET-1-Konzentrationen teilt.

### TABELLE 2

| MARKER bzw. MARKER-KOMBINATION | Cut off (pmol/L) | Spezifität (%) | Sensitivität SKS (%) | Sensitivität LKS mgl AD (%) | Sensitivität ws AD (%) |
|---|---|---|---|---|---|
| MR-proANP | 87,0 pmol/L | 80,0 | 43,1 | 58.7 | 64, 7 |
| MR-proADM | 0,7 nmol/L | 80,0 | 37,3 | 50, 0 | 50, 5 |
| CT-proET1 | 59,5 pmol/L | 80,0 | 49,0 | 52,2 | 45,5 |
| MR-pro-ADM/ CT-proET1 x 1000 | 10,0 | 80,0 | 47,1 | 56,5 | 62.6 |
| MR-pro-ANP/ CT-proET1 | 1,15 | 80, 0 | 68,3 | 76,1 | 80,8 |
| MR-proANP x MR-proADM | 58,5 | 80, 0 | 39,2 | 56,5 | 63,6 |

(fortgesetzt)

| MARKER bzw. MARKER-KOMBINATION | Cut off (pmol/L) | Spezifität (%) | Sensitivität SKS (%) | Sensitivität LKS mgl AD (%) | Sensitivität ws AD (%) |
|---|---|---|---|---|---|
| MR-proANP x MR-pro-ADM/ CT-proET1 x1000 | 0,72 | 80, 0 | 58,8 | 71,7 | 79,8 |

Den Werten der Tabelle 1 bzw. den entsprechenden Figuren ist zu entnehmen, dass insbesondere die MR-proANP-Konzentrationen, jedoch auch die MR-proADM-Konzentrationen, erkennbar an den Werten für die Mediane der verschiedenen Patientengruppen, mit der Schwere der Symptome in der Richtung:

## Kontrollen < SKS < LKS mgl AD ≤ ws AD

klar ansteigen. Es ist ferner zu erkennen, dass die Tendenz, die bereits bei den Einzelbestimmungen von MR-proANP bzw. MR-proADM zu erkennen ist, sich weiter verdeutlicht, wenn man die Messwerte durch die zugehörigen, für jeweils den gleichen Patienten gemessenen CT-proET-1-Konzentrationen teilt.

Tabelle 2 zeigt, dass bei der Bestimmung einzelner Analyten die höchste Sensitivität mit 64,7 % bei der Bestimmung von MR-proANP für die Patientengruppe "wahrscheinlich Alzheimer" (ws AD) erhalten wird, dass die Sensitivität jedoch noch einmal erheblich verbessert wird und für die Gruppe "ws AD" einen Wert von 80,8 % erreicht, wenn man die Messwerte auf die zugehörigen CT-proET-1-Konzentrationen bezieht.

Vorläufige orientierende Bestimmungen der Konzentrationen von BNP (unter Verwendung eines kommerziellen NT-proBNP-Kits der Fa. Roche Diagnostics) bei Patienten der gleichen Patientengruppen und Bezugnahme der erhaltenen Werte auf die patientenspezifischen Messwerte (Konzentrationen in pmol/L) für CT-ET-1 durch Dividieren ergab ähnliche Verbesserungen wie bei der Messung von MR-proANP, d.h auch die Messwerte für BNP wurden durch Dividieren durch die CT-ET-1-Konzentrationen aussagekräftiger.

Bei der Bestimmung von MR-proADM wird bei einem entsprechenden Vorgehen eine ähnliche Verbesserung der Sensitivität erhalten (62,6 % gegenüber 50,5 %).

Die Produktbildung MR-proANP x MR-proADM bringt gegenüber den Werten für den besten Einzelanalyten MR-proANP keine nennenswerte Veränderung, wobei jedoch auch in diesem Falle dann, wenn man das genannte Produkt auf die Messwerte für die CT-proET-1-Konzentrationen bezieht, wie oben eine klare Verbesserung erhält.

Obwohl eine erhöhte Freisetzung vasotroper Peptide, z.B. von ANP, gemessen als MR-proANP Konzentration, oder von ADM, gemessen als MR-proADM, auch bei anderen Erkrankungen (Sepsis; cardiovaskuläre Erkrankungen/ Herzinsuffizienz; diese sind klinisch jedoch in der Regel einfach von Demenzerkrankungen abgrenzbar) messbar ist und vasotrope Peptide daher keine gehirnspezifischen Parameter im engeren Sinne darstellen, eignet sich die Bestimmung vasotroper Peptide, insbesondere als Kombination der Bestimmung von vasodilatorischen Peptiden mit einer gleichzeitigen Bestimmung eines geeigneten vasokonstriktorischen Peptids wie CT-proET-1 zur weiteren Verbesserung der diagnostischen Signifikanz der Bestimmungen, aufgrund der hohen Spezifität und den klar voneinander unterscheidbaren Sensitivitäten sehr gut für Zwecke der Diagnostik von Demenzerkrankungen, insbesondere zur unterstützenden AD-Frühdiagnostik.

Literatur

1. SELKOE D. J. (2001). Alzheimer's disease: genes, proteins, and therapy. Physiological Reviews 81: 741-766

2. Boetsch T., Stübner S. Auer S., Klinisches Bild, Verlauf und Prognose, Kapitel 5 in: Hampel, Padberg, Möller (Hrsg.), Alzheimer Demenz - Klinische Verläufe, diagnostische Möglichkeiten, moderne Therapiestrategien; WVG mbH Stuttgart 2003

3. Boetsch T., Operationalisierte Demenzdiagnostik, Kapitel 6.1 in: Hampel, Padberg, Möller (Hrsg.), Alzheimer Demenz - Klinische Verläufe, diagnostische Möglichkeiten, moderne Therapiestrategien; WVG mbH Stuttgart 2003

4. Reisberg B., Ferris S.H., de Leon M.J., Crook T., 1982, The global deterioration scale for assessment of primary degenerative dementia, Am J Psychiatry 139:1136-1139

5. McKhann G., Drachmann D., Folstein M., Katzman R., Price D., Stadlan E.M. 1984, Clinical diagnosis of Alzheimer's disease: Report of the NINCDS-ARDA work grop under the auspices of departement of health services task force on Alzheimer's disease, Neurology 24: 939-944

6. MCKEITH I. G. (2002). Dementia with lewy bodies. British Journal of Psychiatry 180: 144-147

7. GROWDON J. H., SELKOE D. J., ROSES A., TROJANOWSKI J. Q., DAVIES P., APPEL S. et al. [Working Group Advisory Committee].(1998). Consensus report of the Working Group on Biological Markers of Alzheimer's Disease. [Ronald und Nancy Reagan Institute of the Alzheimer's Association and National Institute on Aging Working Group on Biological Biomarkers of Alzheimer's Disease]. Neurobiology of Aging 19: 109-116

8. FRANK R. A., GALASKO D., HAMPEL H., HARDY J., DE LEON M. J., MEHTA P. D., ROGERS J., SIEMERS E., TROJANOWSKI J. Q. (2003). Biological markers for therapeutic trials in Alzheimer's disease. Proceedings of the biological markers working group; NIA initiative on neuroimaging in Alzheimer's disease. Neurobiology of Aging 24: 521-536

9. TEUNISSEN C. E., DE VENTE J., STEINBUSCH H. W. M., DE BRUIJN C. (2002). Biochemical markers related to Alzheimer's dementia in serum and cerebrospinal fluid. Neurobiology of Aging 23:485-508

10. I.M. KEITH (2000), The Role of Endogenous Lung Neuropeptides in Regulation of the Pulmonary Circulation. Physiol. Res. 49:519-537

11. ALBERTUS BEISHUIZEN, KOEN J. HARTEMINK, ISTVAN VERMES, AB JOHAN GROENEVELD (2005), Circulating cardiovascular markers and mediators in acute illness: an update. Clinica Chimica Acta 354 (2005) 21-34

12. NILS G. MORGENTHALER, JOACHIM STRUCK, BARBARA THOMAS, ANDREAS BERGMANN, Immunoluminometric Assay for the Midregion of Pro-Atrial Natriuretic Peptide in Human Plasma, Clinical Chemistry 50:1, 2004, 234-236.

13. NILS G. MORGENTHALER, JOACHIM STRUCK, CHRISTINE ALONSO, ANDREAS BERGMANN, Measurement of Midregional Proadrenomedullin in Plasma with an Immunoluminometric Assay, Clinical Chemistry 51:10, 2005, 1823-1829

14. JANA PAPASSOTIRIOU, NILS G. MORGENTHALER, JOACHIM STRUCK, CHRISTINE ALONSO, ANDREAS BERGMANN, Immunoluminometric Assay for Measurement of the C-Terminal Endothelin-1 Precursor Fragment in Human Plasma, Clinical Chemistry 52:6, 2006, 1144-1151

15. TARKOWSKI E. (2002). Cytokines in dementias. Current Drug Targets - Inflammation and Allergy 1: 193-200

16. TARKOWSKI E., LILJEROTH A. M., MINTHON L., TARKOWSKI A., WALLIN A., BLENNOW K. (2003). Cerebral pattern of pro- and anti-inflammatory cytokines in dementias. Brain Research Bulletin 61: 255-260

17. CHU D.Q., SMITH D.M., BRAIN S.D. (2001). Studies of the microvascular effects of adrenomedullin and related peptides. Peptides 22:1881-1886

18. Karin Nilsson, Lars Gustavson, Björn Hultberg, Plasma Homocystein Concentration and Its Relation to Symptoms of Vascular Disease in Psychogeriatric Patients, Dement Geriatr Cogn Disord 2005; 20:35-41

19. M.D. Albadalejo, M. Antem, I. Pastor, C. Ruiz, R. Gonzalez-Aniorte, M. Asensio, Determinacion plasmatica de peptidos natriureticosen dementes, Rev Neurol 1997; 25 (139)

20. NILS G. MORGENTHALER, JOACHIM STRUCK, CHRISTINE ALONSO, ANDREAS BERGMANN, Assay for the Measurement of Copeptin, a Stable Peptide Derived from the Precursor of Vasopressin, Clinical Chemistry 52:1, 2006, 112-119

**Patentansprüche**

1. *In vitro* Multiparameter-Verfahren zur Erkennung und Früherkennung, zur Bestimmung des Schweregrads und zur Verlaufsbeurteilung und Prognose von Demenzerkrankungen, die ausgewählt sind aus einer Gruppe, die die Alzheimer Demenz (AD), Demenz mit Lewy-Körperchen (DLB), frontotemporale Demenz (FTD) und verschiedene Formen der vaskulären Demenz (VD) umfasst, bei dem man
in einer Vollblut-, Serum- oder Plasmaprobe einer Person, die an subjektiven oder objektiv nachweisbaren kognitiven Störungen leidet, die Konzentrationen von mindestens zwei vasotropen Peptiden bestimmt, von denen mindestens eines aus einer Gruppe ausgewählt ist, die aus den vasodilatorischen Peptiden ANP, BNP, CNP, Adrenomedullin und dem Peptid CGRP (calcitonin gen related peptide) besteht; und ein weiteres aus der Gruppe der vasokonstriktorischen Endothelin-peptide ausgewählt ist, insbesondere Endothelin 1 ist,
die erhaltenen personenspezifischen Messwerte für die jeweiligen Konzentrationen der mindestens zwei bestimmten vasotropen Peptide rechnerisch zu einem personenspezifischen komplexen Bezugswert kombiniert und
auf der Basis des ermittelten personenspezifischen komplexen Bezugswerts Schlüsse hinsichtlich des Vorliegens einer Demenzerkrankung bei der Person, oder einer für diese Erkrankung typischen Frühform, oder hinsichtlich des Verlaufs der Erkrankung und/oder des Erfolgs der Bemühungen zu ihrer Abmilderung oder Verhinderung zieht.

2. Verfahren nach Anspruch 1, **dadurch gekennzeichnet, dass** man bei der rechnerischen Auswertung pexionenspezifische Mesewerte für Konzentrationen der vasodilatorischen Peptide durch Multiplikation kombiniert und personenspezifische Messwerte für Konzentrationen der Endothelin-Peptide durch Division kombiniert.

3. Verfahren nach Anspruch 1, **dadurch gekennzeichnet**, das man die Konzentration von ANP als Konzentration eines pro-ANP-Fragments mit Hilfe eines Assays bestimmt, der einen mittleren Abschnitt des proANP (MR-proANP) erkennt.

4. Verfahren nach Anspruch 1, **dadurch gekennzeichnet, dass** man die Konzentration von Adrenomedullin (ADM) als Konzentration eines midregionalen proADM-Fragments (MR-proADM) bestimmt, das die Aminosäuren 45-92 des Prä-Proadrenomedullins umfasst.

5. Verfahren nach Anspruch 1, **dadurch gekennzeichnet, dass** man die Konzentration von Endothelin 1 (ET-1) als Konzentration eines C-terminalen ET-1-Fragments (CT-proET-1) bestimmt, das die Aminosäuren 168-212 des Prä-Proendothelins 1 umfasst.

6. Verfahren nach einem der Ansprüche 1 bis 5, **dadurch gekennzeichnet, dass** man die Bestimmung der mindestens zwei vasotropen Peptide mit Hilfe immundiagnostischer Bestimmungsverfahren in Form von Immunoassays vom Sandwichtyp durchführt.

7. Verfahren nach einem der Ansprüche 1 bis 6, **dadurch gekennzeichnet, dass** man zur Ermittlung des patentientenspezifischen Bezugswerts den Messwert für die Konzentration eines der vasodilatorischen Peptide, oder den durch rechnerische Multiplikation von wenigstens zwei Messwerten für die Konzentrationen von wenigstens zwei vasodilatorischen Peptiden erhaltenen Wert, durch die Konzentration für das vasokonstriktorische Peptid ET-1 teilt.

8. Verfahren nach einem der Ansprüche 1 bis 7, **dadurch gekennzeichnet, dass** es im Rahmen der Alzheimer-Diagnostik zur Erkennung von Frühformen der Alzheimer Demenz (AD) durchgeführt wird.

9. Verfahren nach einem der Ansprüche 1 bis 8, **dadurch gekennzeichnet, dass** die Multiparameter-Bestimmung als Simultanbestimmung mittels einer Chiptechnologie-Messvorrichtung oder einer immunchromatographischen Meßvorrichtung erfolgt.

10. Verfahren nach Anspruch einem der Ansprüche 1 bis 9, **dadurch gekennzeichnet, dass** die Ermittlung und Auswertung des komplexen Messergebnisses der Multiparameter-Bestimmung mit Hilfe eines Computerprogramms erfolgt.


**Claims**

1. *In vitro* multiparameter method for the detection and early detection, for the determination of the severity and for the assessment of the course and prognosis of neurodegenerative diseases which are selected from the group consisting of Alzheimer's disease (AD), dementia with Lewy bodies (DLB), frontotemporal dementia (FTD) and various forms of vascular dementia (VD), in which

in a whole blood, serum or plasma sample of a person who is suffering from subjective or objectively detectable cognitive disturbances the concentrations of at least two vasotropic peptides are determined, at least one of them being selected from the vasodilatory peptides ANP, BNP, CNP, adrenomedullin and the peptide CGRP (calcitonin gene related peptide), and another one being selected from the group of vasoconstrictive peptides, especially being endothelin 1,

the person-specific measured values obtained for the respective concentrations of the at least two vasotropic peptides determined are combined computationally to give a person-specific complex reference value and

conclusions regarding the presence of a neurodegenerative disease in the person, or of an early form typical of said disease, or regarding the course of the disease and/or the success of the efforts to alleviate or prevent it, are drawn on the basis of the person-specific complex reference value determined.

2. Method according to Claim 1, **characterized in that**, in the computational evaluation, person-specific measured values for concentrations of the vasotropic peptides are combined by multiplication, and person-specific measured values for concentrations of endothelin peptides are combined by division.

3. Method according to Claim 1, **characterized in that** the concentration of ANP is determined as the concentration of a proANP fragment with the aid of an assay which detects a midregional segment of proANP (MR-proANP) .

4. Method according to Claim 1, **characterized in that** the concentration of adrenomedullin (ADM) is determined as the concentration of a midregional proADM fragment (MR-proADM) which comprises the amino acids 45-92 of pre-proadrenomedullin.

5. Method according to Claim 1, **characterized in that** the concentration of endothelin 1 (ET-1) is determined as the concentration of a C-terminal ET-1 fragment (CT-proET-1) which comprises the amino acids 168-212 of pre-proendothelin 1.

6. Method according to any of Claims 1 to 5, **characterized in that** the determination of the at least two vasotropic peptides is carried out with the aid of immunodiagnostic assay methods in the form of immunoassays of the sandwich type.

7. Method according to any of Claims 1 to 6, **characterized in that**, for determining the patient-specific reference value, the measured value for the concentration of one of the vasodilatory peptides, or the value obtained by computational multiplication of at least two measured values for the concentrations of at least two vasodilatory peptides, is divided by the concentration for the vasoconstrictive peptide ET-1.

8. Method according to Claim 7, **characterized in that** it is carried out as part of Alzheimer's diagnosis for detecting early forms of Alzheimer's disease (AD).

9. Method according to any of Claims 1 to 8, **characterized in that** the multiparameter determination is effected as a simultaneous determination by means of a chip technology measuring apparatus or an immunochromatographic measuring apparatus.

10. Method according to any of Claims 1 to 9, **characterized in that** the determination and evaluation of the complex measured result of the multiparameter determination is effected with the aid of a computer program.

## Revendications

1. Procédé in vitro multiparamètre pour le dépistage et le diagnostic précoce, pour la détermination du degré de gravité et pour l'évaluation de l'évolution et le pronostic de maladies démentielles, qui sont choisies dans le groupe comprenant la maladie de Alzheimer (AD), la démence à corps de Lewy (DLB), la démence frontotemporale (FTD) et différentes formes de la démence vasculaire (VD), procédé dans lequel

   - on détermine, dans un échantillon de sang complet, de sérum ou de plasma d'une personne, qui souffre de perturbations cognitives, pouvant être identifiées subjectivement ou objectivement, la concentration d'au moins deux peptides vasotropes, dont l'un, au moins, est choisi dans le groupe comprenant les peptides vasodilateurs ANP, BNP, CNP, l'adrénomédulline et le peptide CGRP (calcitonin gen related peptide), et un autre est choisi dans le groupe des peptides d'endothéline en particulier l'endothéline 1,
- on combine par calcul les valeurs de mesure spécifiques à la personne, ayant été obtenues, pour les concentrations respectives des au moins deux peptides vasotropes en une valeur de référence complexe, spécifiques à la personne, et
- on tire, sur la base des valeurs de mesure spécifiques, obtenues, des conclusions au sujet de la maladie démentielle, présente chez la personne, ou d'une forme précoce, typique pour cette maladie, ou au sujet de l'évolution de la maladie et / ou du succès des mesures prises pour l'atténuer ou l'éviter.

2. Procédé selon la revendication 1, **caractérisé en ce que**, lors de l'évaluation mathématique, on combine par multiplication des valeurs spécifiques à la personne, pour des concentrations des peptides vasodilateurs, et combine par division des valeurs spécifiques à la personne pour des concentrations des peptides d'endothéline.

3. Procédé selon la revendication 1, **caractérisé en ce que** l'on détermine la concentration d'ANP en tant que concentration d'un fragment pro-ANP, à l'aide d'un essai, qui identifie une section moyenne du proANP (MR - proANP).

4. Procédé selon la revendication 1, **caractérisé en ce que** l'on détermine la concentration d'adrénomédulline (ADM) en tant que concentration d'un fragment pro-ADM à région moyenne (MR - proADM), qui comprend les acides aminés 45 - 92 de pré-proendothéline 1.

5. Procédé selon la revendication 1, **caractérisé en ce que** l'on détermine la concentration d'endothéline 1 (ET - 1) en tant que concentration d'un fragment ET-1 C - terminal (CT - pro-ET - 1), qui comprend les acides aminés 168 - 212 de pré-proendothéline 1.

6. Procédé selon l'une des revendications 1 à 5, **caractérisé en ce que** l'on effectue la détermination des au moins deux peptides vasotropes à l'aide de procédés de détermination immunodiagnostiques, sous la forme d'immunoessais de type sandwich.

7. Procédé selon l'une des revendications 1 à 6, **caractérisé en ce que**, pour la détermination de la valeur de référence spécifique au patient, on divise la valeur de mesure pour la concentration de l'un des peptides vasodilateurs ou la valeur obtenue par multiplication d'au moins deux valeurs de mesure pour les concentration d'au moins deux peptides vasodilateurs, par la concentration pour le peptide vasoconstricteur ET-1.

8. Procédé selon l'une des revendications 1 à 7, **caractérisé en ce que** celui-ci est effectué, dans le cadre du diagnostic de la maladie d'Alzheimer, pour le dépistage de formes précoces de la démence d'Alzheimer (AD).

9. Procédé selon l'une des revendications 1 à 8, **caractérisé en ce que** la détermination multiparamètre est effectuée au moyen d'un dispositif de mesure selon la technologie des microplaquettes ou d'un dispositif immunochromatographique.

10. Procédé selon l'une des revendications 1 à 9, **caractérisé en ce que** la détermination et l'évaluation du résultat complexe de la détermination multiparamètre est effectuée à l'aide d'un programme d'ordinateur.

FIGURE 1

FIGURE 2

FIGURE 3

FIGURE 4

FIGUR 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1562984 B1
- EP 1488209 B1
- WO 2004090546 A
- WO 1564558 B1
- WO 2005078456 A

- DE 102005036094
- DE 102006023175
- WO 2006018315 A
- WO 2004046181 A

**In der Beschreibung aufgeführte Nicht-Patentliteratur**

- **SELKOE D. J.** Alzheimer's disease: genes, proteins, and therapy. *Physiological Reviews,* 2001, vol. 81, 741-766
- Verlauf und Prognose. **BOETSCH T. ; STÜBNER S. ; AUER S. ; KLINISCHES BILD.** Alzheimer Demenz - Klinische Verläufe, diagnostische Möglichkeiten, moderne Therapiestrategien. 2003
- Operationalisierte Demenzdiagnostik. **BOETSCH T.** Alzheimer Demenz - Klinische Verläufe, diagnostische Möglichkeiten, moderne Therapiestrategien. 2003
- **REISBERG B. ; FERRIS S.H. ; DE LEON M.J. ; CROOK T.** The global deterioration scale for assessment of primary degenerative dementia. *Am J Psychiatry,* 1982, vol. 139, 1136-1139
- **MCKHANN G. ; DRACHMANN D. ; FOLSTEIN M. ; KATZMAN R. ; PRICE D. ; STADLAN E.M.** Clinical diagnosis of Alzheimer's disease: Report of the NINCDS-ARDA work grop under the auspices of departement of health services task force on Alzheimer's disease. *Neurology,* 1984, vol. 24, 939-944
- **MCKEITH I. G.** Dementia with lewy bodies. *British Journal of Psychiatry,* 2002, vol. 180, 144-147
- Consensus report of the Working Group on Biological Markers of Alzheimer's Disease. [Ronald und Nancy Reagan Institute of the Alzheimer's Association and National Institute on Aging Working Group on Biological Biomarkers of Alzheimer's Disease. **GROWDON J. H. ; SELKOE D. J. ; ROSES A. ; TROJANOWSKI J. Q. ; DAVIES P. ; APPEL S. et al.** Neurobiology of Aging. 1998, vol. 19, 109-116
- **FRANK R. A. ; GALASKO D. ; HAMPEL H. ; HARDY J. ; DE LEON M. J. ; MEHTA P. D. ; ROGERS J. ; SIEMERS E. ; TROJANOWSKI J. Q.** Biological markers for therapeutic trials in Alzheimer's disease. Proceedings of the biological markers working group; NIA initiative on neuroimaging in Alzheimer's disease. *Neurobiology of Aging,* 2003, vol. 24, 521-536

- **TEUNISSEN C. E. ; DE VENTE J. ; STEINBUSCH H. W. M. ; DE BRUIJN C.** Biochemical markers related to Alzheimer's dementia in serum and cerebrospinal fluid. *Neurobiology of Aging,* 2002, vol. 23, 485-508
- **I.M. KEITH.** The Role of Endogenous Lung Neuropeptides in Regulation of the Pulmonary Circulation. *Physiol. Res.,* 2000, vol. 49, 519-537
- **ALBERTUS BEISHUIZEN ; KOEN J. HARTEMINK ; ISTVAN VERMES ; AB JOHAN GROENEVELD.** Circulating cardiovascular markers and mediators in acute illness: an update. *Clinica Chimica Acta,* 2005, vol. 354, 21-34
- **NILS G. MORGENTHALER ; JOACHIM STRUCK ; BARBARA THOMAS ; ANDREAS BERGMANN.** Immunoluminometric Assay for the Midregion of Pro-Atrial Natriuretic Peptide in Human Plasma. *Clinical Chemistry,* 2004, vol. 50 (1), 234-236
- **NILS G. MORGENTHALER ; JOACHIM STRUCK ; CHRISTINE ALONSO ; ANDREAS BERGMANN.** Measurement of Midregional Proadrenomedullin in Plasma with an Immunoluminometric Assay. *Clinical Chemistry,* 2005, vol. 51 (10), 1823-1829
- **JANA PAPASSOTIRIOU ; NILS G. MORGENTHALER ; JOACHIM STRUCK ; CHRISTINE ALONSO ; ANDREAS BERGMANN.** Immunoluminometric Assay for Measurement of the C-Terminal Endothelin-1 Precursor Fragment in Human Plasma. *Clinical Chemistry,* 2006, vol. 52 (6), 1144-1151
- **TARKOWSKI E.** Cytokines in dementias. *Current Drug Targets - Inflammation and Allergy,* 2002, vol. 1, 193-200
- **TARKOWSKI E. ; LILJEROTH A. M. ; MINTHON L. ; TARKOWSKI A. ; WALLIN A. ; BLENNOW K.** Cerebral pattern of pro- and anti-inflammatory cytokines in dementias. *Brain Research Bulletin,* 2003, vol. 61, 255-260

- **CHU D.Q. ; SMITH D.M. ; BRAIN S.D.** Studies of the microvascular effects of adrenomedullin and related peptides. *Peptides,* 2001, vol. 22, 1881-1886
- **KARIN NILSSON ; LARS GUSTAVSON ; BJÖRN HULTBERG.** Plasma Homocystein Concentration and Its Relation to Symptoms of Vascular Disease in Psychogeriatric Patients. *Dement Geriatr Cogn Disord,* 2005, vol. 20, 35-41
- **M.D. ALBADALEJO ; M. ANTEM ; I. PASTOR ; C. RUIZ ; R. GONZALEZ-ANIORTE ; M. ASENSIO.** Determinacion plasmatica de peptidos natriureticosen dementes. *Rev Neurol,* 1997, vol. 25 (139
- **NILS G. MORGENTHALER ; JOACHIM STRUCK ; CHRISTINE ALONSO ; ANDREAS BERGMANN.** Assay for the Measurement of Copeptin, a Stable Peptide Derived from the Precursor of Vasopressin. *Clinical Chemistry,* 2006, vol. 52 (1), 112-119